# EUROPEAN PATENT APPLICATION

(11) **EP 2 070 899 A1**
(43) Date of publication of application: **17.06.2009**
(21) Application number: 08170637.6
(22) Date of filing: 04.12.2008
(51) Int. Cl.: C07C 19/08, C07D 209/42, C07D 403/04

(54) **Deprotection of N-BOC compounds**

(30) Priority: 14.12.2007 US 13931 P
(71) Applicant: F. Hoffmann-La Roche AG, 4070 Basel (CH)
(72) Inventor: Choy, Jason Chi-Chung, Alameda, CA 94501 (US); Jaime-Figueroa, Saul, Fremont, CA 94555 (US); Muchowski, Joseph M., Westbank British Columbia (CA); Wagner, Paul J., Mountain View, CA 94040 (US)
(74) Representative: Küng, Peter

(57) **Abstract**

Organic compounds having nitrogen atoms protected with t-butoxycarbonyl are effectively deprotected by heating in a fluorinated alcohol solution.

## Description

This invention relates generally to the field of synthetic chemistry. More particularly, the invention relates to methods for deprotecting N-BOC protected organic compounds using fluorinated alcohols.

Among various nitrogen protecting groups in organic chemistry, the t-butoxycarbonyl (BOC) group is perhaps one of the most widely used due to its exceptional stability towards a variety of reagents and reaction conditions (T.W. Greene & P.G.M. Wuts, "Protective Groups in Organic Synthesis", 3rd ed., (1999) John Wiley and Sons, New York; A. Ganesan et al., Mol. Divers. (2005) 9:291-93). As a result, removal of the BOC group remains of prime importance in organic synthesis. Cleavage of BOC on nitrogen is generally achieved under acidic conditions (Greene & Wuts, supra), however, basic, thermolytic, and microwave assisted conditions are also described in the literature (M. Chakrabarty & T. Kundu, Synth. Comm. (2006) 36:2069-77; J.N. Tom et al, Tet. Lett. (2004) 45:905-06; V.H. Rawal and M.P. Cava, Tet. Lett. (1985) 26(50):6141-42; J.G. Siro et al., Synlett (1998) 147-48).

We have now invented a method for removing BOC protecting groups from nitrogen atoms in organic compounds using fluorinated alcohols. The reaction conditions are neutral and do not require additional reagents (apart from solvents). Thus, the product is recovered by a simple solvent evaporation without any work up and in some cases, no further purification is needed.

One aspect of the invention is a method for removing a BOC protecting group from a nitrogen atom, comprising dissolving an N-BOC protected compound in a fluorinated alcohol, and heating the solution to remove BOC from the BOC-protected nitrogen atom.

Another aspect of the invention is a method for sequentially removing BOC protecting groups from nitrogen atoms having different lability in the same compound using first and second fluorinated alcohol solvents having different reactivities, comprising dissolving a compound comprising first and second BOC-protected nitrogen atoms having different lability in a first fluorinated alcohol to form a first solution; heating the first solution to remove BOC from said first BOC-protected nitrogen atom, providing a partially-deprotected compound; dissolving said partially-deprotected compound in a second fluorinated alcohol solvent having higher reactivity than said first fluorinated alcohol solvent, to form a second solution; and heating said second solution to remove BOC from said second BOC-protected nitrogen atom.

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

The term "BOC" refers to the radical t-butoxycarbonyl, (CH3)3CC(O)O-.

The terms "BOC-protected nitrogen" and "N-BOC" refer to a nitrogen atom to which a BOC radical is covalently bound. Similarly, "BOC-protected compound" refers to an organic compound that comprises a BOC-protected nitrogen.

The term "deprotected compound" refers to a compound from which BOC has been removed from a BOC-protected nitrogen. Note that a deprotected compound within the scope of this invention may still retain other protecting groups, which are generally undisturbed by the method of the invention.

The term "fluorinated alcohol" refers to compounds of the formula R1R2R3C-OH, where R1 is a fluorinated lower alkyl radical, and R2 and R3 are each independently H or a fluorinated lower alkyl radical. Exemplary fluorinated alcohols include, without limitation, 2,2,2-trifluoroethanol ("TFE"), 1,1,1,3,3,3-hexafluoroisopropanol ("HFIP"), 3,3,4,4,4-pentafluorobutan-2-ol ("PFB"), and the like.

The term "lower alkyl" refers to monovalent hydrocarbon radicals composed of carbon and hydrogen, and having no unsaturation. Lower alkyl radicals may be straight or branched, and contain from 1 to 6 carbon atoms, inclusive.

The term "fluorinated lower alkyl" refers to a lower alkyl radical in which one or more hydrogen atoms has been replaced by fluorine. Exemplary fluorinated lower alkyl radicals include, without limitation, CF₃-, CHF₂-, CF₃CF₂-, CHF₂CF₂-, and the like.

The term "labile" as used herein refers to the relative bond strength and ease of removing the BOC protecting group.

All patents and publications identified herein are incorporated herein by reference in their entirety.

The invention provides a new, practical method to cleanly deprotect BOC-nitrogens using a fluorinated alcohol such as 2,2,2-trifluoroethanol (TFE) or hexafluoroisopropanol (HFIP) as a solvent, in quantitative yields.

In practice, an N-BOC protected compound is first dissolved in a fluorinated alcohol such as TFE or HFIP. The quantity of fluorinated alcohol required to dissolve the protected compound will depend in general on the solubility of the compound. As a starting point, one may begin with a ratio of about 1 mmol protected compound to about 5 mL of fluorinated alcohol, and adjust the ratio by routine experimentation to maximize results. If the protected compound is not sufficiently soluble in a fluorinated alcohol, a co-solvent such as benzene, toluene, pyridine, dimethylsulfoxide, N-methylpyrrolidine, dichloromethane, chloroform, dioxane, tetrahydrofuran, or the like may be added.

The solution may be heated by convention methods, for example by gas burner, oil bath, and the like. Preferably, the solution is heated using a microwave radiator, such as a Biotage INITIATOR^{™} 60 focused microwave reactor. The solution is preferably stirred during heating.

In general, the reaction times and temperatures necessary will depend upon the nature of the compound to be deprotected and the heating method. When using TFE or HFIP with most protected compounds and conventional heating at the reflux temperature of the solvent, a reaction time of about 30 minutes to about 48 hours is generally necessary. When using TFE or HFIP with most protected compounds and microwave heating, a temperature of between about 80°C and about 200°C is sufficient, preferably between about 100°C and 170°C. Reaction times may range, in general, from about 1 minute to about 6 hours, typically from about 1 hour to about 4 hours. Optimal reaction times and selection of fluorinated alcohol are determined by routine experimentation, for example following the Examples set forth below. In general, BOC groups that are less labile can be removed by (a) increasing the reaction time, (b) switching to a more reactive fluorinated alcohol (for example, from TFE to HFIP), and/or (c) increasing the temperature.

After completion of the deprotection reaction, the fluorinated alcohol may be removed by evaporation, and the deprotected compound recovered and purified by convention methods, for example, by column chromatography, HPLC, recrystallization, and the like. The fluorinated alcohol is preferably recovered and reused.

In cases in which a given compound has multiple different nitrogen atoms, having different labilities, it is possible to deprotect BOC-protected nitrogen atoms sequentially, proceeding with the most labile nitrogen. For example, in compounds having an aromatic nitrogen and an aliphatic nitrogen, it is possible to remove the BOC group from the aromatic nitrogen without disturbing the aliphatic nitrogen BOC, and to then remove the BOC group from the aliphatic nitrogen in a separate step. This permits one to modify the aromatic nitrogen without simultaneously modifying the aliphatic nitrogen. To effect such sequential deprotection, one removes the first (most labile) BOC using the least reactive fluorinated alcohol (for example, TFE). Routine experimentation may be necessary to determine the optimal choice of fluorinated alcohol, reaction temperature, and reaction time. As a starting point, an aromatic nitrogen may be deprotected in the presence of a BOC-protected aliphatic nitrogen by using TFE for 2 hours at 150°C in a microwave reactor. After the first BOC has been removed, the aromatic amine may be modified or derivatized while the aliphatic N-BOC remains in place. When the aliphatic nitrogen no longer requires protection, the BOC group can be removed using a more reactive fluorinated alcohol such as HFIP (for example, for 2 hours at 150°C in a microwave reactor).

### Example 1

(A) A solution of the N-Boc protected amine (1 mmol) in TFE (2,2,2-trifluoroethanol) or HFIP (hexafluoroisopropanol) (5 mL) was placed in a sealed microwave vial. The reaction mixture was heated (100°C or 150°C) in a Biotage - Initiator^{™} Sixty microwave reactor with stirring until the disappearance of the starting material was observed. After cooling to room temperature, the mixture was evaporated to dryness under reduced pressure. The crude product was purified by flash-column chromatography. ¹H NMR and ¹³C NMR were measured on Bruker Avance DPX-300 NMR or Bruker Avance-300 NMR spectrometers, operating at a proton (¹H) frequency of 300.13 MHz and carbon (¹³C) frequency of 75.43 MHz.
(B) 3-Benzyl-5-methyl-2,4-dioxo-3,4-dihydro-2H-pyrimidine-1-BOC (1 mmol) was deprotected by (a) heating at reflux for 1 h in TFE (5 mL), or (b) heating in TFE (5 mL) for 5 min at 100°C in a microwave reactor, and purified by flash-column chromatography to provide 3-benzyl-5-methyl-1H-pyrimidine-2,4-dione in 83% yield (a) and 97% yield (b). Product mp 207-208°C; ¹H NMR (DMSO-d6) 11.00 (broad s,1H), 7.35 (s,1H), 7.22-7.34 (m,5H), 4.97 (s,2H), 1.80 (s,3H,); ¹³C NMR (DMSO-d6) 164.15, 151.74, 137.81, 136.97, 128.65, 127.94, 127.41, 107.62, 43.20, 12.85; MS ESI: m/z (%) 217 (M+H+, 100); Anal. calc. for C₁₂H₁₂N₂O₂: C,66.64; H,5.59; N,12.95. Found: C,66.66; H,5.46; N,13.01.
(C) N-BOC-2-formyl-pyrrole (1 mmol) was deprotected by (a) heating at reflux for 6 h in TFE (5 mL), or (b) heating in TFE (5 mL) for 30 min at 100°C in a microwave reactor, and purified by flash-column chromatography to provide 1H-pyrrole-2-carbaldehyde in 65% yield (a) and 91% yield (b). Product mp = 44-45°C; ¹H NMR (CDCl₃) 9.56 - 9.86 (broad s, 1H), 9.54 (s, 1H), 7.15 (s, 1H), 6.92 - 7.05 (m, 1H), 6.27 - 6.43 (m, 1H); ¹³C NMR (CDCl₃) 180.49, 132.92, 126.44, 121.40, 111.36. MS ESI: m/z (%) 96 (M+H+, 100); HRMS ESI m/z 96.04388 (M+H+). Calculated 96.04439.
(D) 5-Chloro-(1-BOC)-1H-indole-3-carboxylic acid amide (1 mmol) was deprotected by (a) heating at reflux for 12 h in TFE (5 mL), or (b) heating in TFE (5 mL) for 1 h at 100°C in a microwave reactor, and purified by flash-column chromatography, to provide 5-chloro-1H-indole-3-carboxylic acid amide in 99% yield (a) and 98% yield (b). Product mp 248-249°C; ¹H NMR (DMSO-d6) 11.72 (broad s, 1 H), 8.15 (d, J=2.26 Hz, 1 H), 8.09 (d, J=3.01 Hz, 1 H), 7.60-7.40 (broad s, 1H), 7.45 (d, J=8.67 Hz, 1 H), 7.15 (dd, J=8.48, 2.07 Hz, 1 H), 7.00-6.75 (broad s, 1 H); ¹³C NMR (DMSO-d6) 166.42, 134.99, 130.17, 127.83, 125.43, 122.15, 120.55, 113.74, 110.54; MS ESI: m/z (%) 195 (M+H+, 100); HRMS ESI m/z (M+H+) 195.03188. Calculated 195.03197.
(E) N-BOC-4-chlorophenylamine (1 mmol) was deprotected by (a) heating at reflux for 36 h in HFIP (5 mL), or (b) heating in HFIP (5 mL) for 1 h at 150°C in a microwave reactor, and purified by flash-column chromatography, to provide 4-chlorophenylamine in 81% yield (a) and 80% yield (b). Product mp 71-72°C; ¹H NMR (CDCl₃), 7.04 - 7.16 (m, 2H), 6.54 - 6.67 (m, 2H), 3.65 (broad s., 2H); ¹³C NMR (CDCl₃) 144.92, 129.10, 123.14, 116.21; MS ESI m/z (%) 128 (M+H+,100%); HRMS ESI m/z (M+H+) 128.02576. Calc = 128.02615.
(F) N-BOC-2-(2,6-dimethylphenoxy)-1-methylethylamine (1 mmol) was deprotected by heating at 150°C for 2 h in HFIP in a microwave reactor, and purified by flash-column chromatography to provide 2-(2,6-dimethylphenoxy)-1-methylethylamine in 81% yield. Product: Oil; ¹H NMR (CDCl₃), 7.06 - 6.86 (m, 3H); 3.59 - 3.51 (m, 1H), 3.70 - 3.62 (m, 1H), 3.46 - 3.29 (m, 1H), 2.30 (s, 6H), 1.72 (broad s, 2H), 1.18 (d, J=6.78 Hz, 3H); ¹³C NMR (CDCl₃) 155.49, 130.81, 128.88, 123.83, 78.23, 47.29, 19.78, 16.32; MS ESI m/z (%) 180 (M+H+,100%); HRMS ESI m/z (M+H+) 180.13782. Calc 180.13829.

### Example 2

Following the procedure set forth in Example 1(A) above, indole derivatives were deprotected using TFE or HFIP in a microwave reactor at 150°C as set forth in Table 1 below.

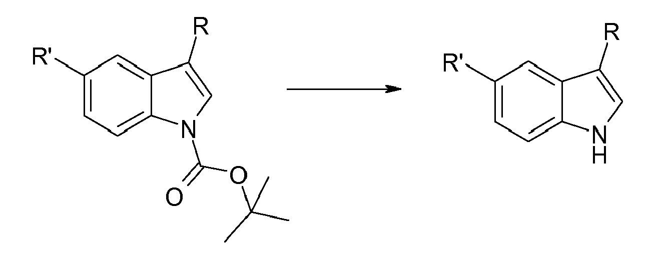

**Table 1: Deprotection of Indoles**

| **Compound No.** | **R** | **R'** | **Fluorinated alcohol** | **Time** | **Yield^{a}** |
|---|---|---|---|---|---|
| 1 | -CONH₂ | Cl | TFE | 5 min | 98% |
| 1 | -CONH₂ | Cl | HFIP | 5 min | 97% |
| 2 | H | H | TFE | 15 min | 99% |
| 2 | H | H | HFIP | 5 min | 97% |
| 3 | -CHO | H | TFE | 5 min | 91% |
| 3 | -CHO | H | HFIP | 5 min | 96% |
| 4 | H | Cl | TFE | 1h | 98% |
| 4 | H | Cl | HFIP | 5 min | 94% |
| 5 | H | -OMe | TFE | 1 h | 95% |
| 5 | H | -OMe | HFIP | 15 min | 98% |
| 6 | H | -CN | TFE | 1 h | 98% |
| 6 | H | -CN | HFIP | 5 min | 98% |
| 7 | H | -NO₂ | TFE | 15 min | 91% |
| 7 | H | -NO₂ | HFIP | 5 min | 99% |
| 8 | H | -NH₂ | TFE | 15 min | 81% |
| 8 | H | -NH₂ | HFIP | 15 min | 70% |

| | | | | | |
|---|---|---|---|---|---|
| ^{a} yield after chromatography | | | | | |

### Product data:

Compound 1: mp 248-249°C; ¹H NMR (DMSO-d6) 11.72 (broad s, 1 H), 8.15 (d, J=2.26 Hz, 1 H), 8.09 (d, J=3.01 Hz, 1 H), 7.60-7.40 (broad s, 1H), 7.45 (d, J=8.67 Hz, 1 H), 7.15 (dd, J=8.48, 2.07 Hz, 1 H), 7.00-6.75 (broad s, 1 H); ¹³C NMR (DMSO-d6) 166.42, 134.99, 130.17, 127.83, 125.43, 122.15, 120.55, 113.74, 110.54; MS ESI: m/z (%) 195 (M+H+, 100); HRMS ESI m/z (M+H+) 195.03188. Calc 195.03197.
Compound 2: mp 50-51°C; ¹H NMR (CDCl₃) s 8.08 (broad s, 1H), 7.65 (dd, 1H), 7.38 (m, 2H), 7.23-7.09 (m, 2H), 6.56-6.54 (m, 1H); ¹³C NMR (CHCl₃-d) 135.74, 127.82, 124.10, 121.97, 120.72,119.80, 111.00, 102.61; MS EI: m/z (%) 117 (M+,100).
Compound 3: mp 196-197°C; ¹H NMR (DMSO-d6) 12.14 (broad s, 1H), 9.95 (s, 1H), 8.30-8.09 (m, 2H), 7.56-7.20 (m, 3H); ¹³C NMR (DMSO-d6)185.34, 138.85, 137.43, 124.49, 123.84, 122.50m 121.20, 118.54, 112.80; MS ESI: m/z (%) 146 (M+H+; 100); HRMS ESI m/z (M+H+) 146.05963. Calc 146.06004.
Compound 4: mp 74-75°C; ¹H NMR (CDCl₃), 8.13 (broad s, 1H, D₂O exch.), 7.61 (s, 1H), 7.31-7.12 (m, 3H), 6.50-6.48 (m, 1H); ¹³C NMR (CDCl₃) 134.11, 128.94, 125.51, 125.46, 122.31, 120.11, 111.97, 102.41; MS EI: m/z (%) 151 (M+,100%); HRMS ESI m/z (M+H+) 152.02585. Calc 152.02615.
Compound 5: mp 55-56°C; ¹H NMR (CDCl₃), 8.03 (broad s, 1H, D₂O exch.),7.28-6.84 (m, 4H), 6.49-6.47 (m, 1H), 3.85 (s, 3H); ¹³C NMR(CDCl₃) 154.19, 130.94, 128.27, 124.85, 112.35, 111.70, 102.38, 102.30, 55.85; MS ESI: m/z (%) 148 (M+H+,100%); HRMS ESI m/z (M+H+) 148.07531. Calc 148.07569.
Compound 6: mp 102-104°C; ¹H NMR (CDCl₃), 8.68 (broad s, 1H, D2O exch.), 8.00-7.99 (m, 1H), 7.49-7.40 (m, 2H), 7.36-7.34 (m,1H), 6.64-6.62 (m, 1H); ¹³C NMR (CDCl₃) 137.50, 127.66, 126.48, 126.41, 124.87, 120.87, 112.02, 103.43, 102.79; MS EI: m/z (%) 142 (M+,100%); HRMS ESI m/z (M+H+) 143.06006. Calc 143.06037.
Compound 7: mp 141-142°C; ¹H NMR (CDCl₃), 8.62 (d, 1H), 8.60-8.45 (broad s, 1H, D₂O exch.), 8.15-8.10 (m, 1H), 7.46-7.37 (m, 2H), 6.76-6.74 (m, 1H); ¹³C NMR (CDCl₃) 141.20, 139.03, 127.75, 126.95, 117.57, 116.83, 111.10, 104.05; MS EI: m/z (%) 162 (M+,100%); Anal. calc. for C8H6N2O2: C, 59.26; H, 3.73; N, 17.28. Found: C, 59.11; H, 3.46; N, 17.14.
Compound 8: mp 129-130°C; ¹H NMR (CDCl₃), 7.98 (d, 1H), 7.21 (d, 1H), 7.14 (t, 1H), 6.96 (d, 1H), 6.68 (dd, 1H), 6.39 (s, 1H), 3.51 (broad s, 2H); ¹³C NMR (CDCl₃) 139.54, 130.63, 128.77, 124.69, 112.95, 111.49, 105.52, 101.56; MS ESI m/z (%) 133 (M+H+,100%); HRMS ESI m/z (M+H+) 133.07560. Calc 133.07602.

### Example 3

The deprotection of a series of anilines was then explored using the protocol set forth in Example 2 above. The results are summarized in Table 2 below. Anilines with electronwithdrawing substituents were found to react faster than those with electron-donating groups. The reaction conditions employed were found to be compatible with other protecting groups such as -NCbz, -NAlloc and -OTIPS.

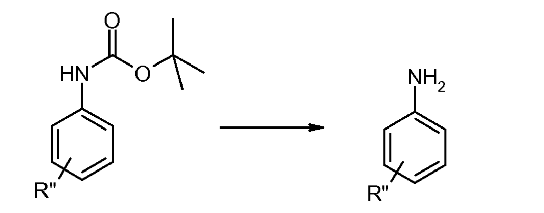

**Table 2: Deprotection of Anilines**

| **Compound No.** | **R"** | **Fluorinated alcohol** | **Time** | **Yield^{a}** |
|---|---|---|---|---|
| 9 | 4-Cl | TFE | 1.5 h | 98% |
| 9 | 4-Cl | HFIP | 1 h | 80% |
| 10 | 4-Br, 3-Cl | TFE | 1 h | 97% |
| 10 | 4-Br, 3-Cl | HFIP | 15 min | 77% |
| 11 | 4-NO₂ | TFE | 1 h | 95% |
| 11 | 4-NO₂ | HFIP | 5 min | 76% |
| 12 | 4-OMe | TFE | 2 h | 95% |
| 12 | 4-OMe | HFIP | 0.5 h | 85% |
| 13 | 3-OMe | TFE | 1 h | 95% |
| 13 | 3-OMe | HFIP | 0.5 h | 89% |
| 14 | 2-I | TFE | 1 h | 98% |
| 14 | 2-I | HFIP | 1 h | (decomposed) |
| 15 | 4-NH₂ | TFE | 3 h | 93% |
| 15 | 4-NH₂ | HFIP | 0.5 h | 97% |
| 16 | 3-OTIPS | TFE | 2 h | 81% |
| 16 | 3-OTIPS | HFIP | 0.5 h | 81% |
| 17 | 4-NHCbz | TFE | 2 h | 99% |
| 17 | 4-NHCbz | HFIP | 2 h | 86% |
| 18 | 4-NHAlloc | TFE | 1 h | 98% |
| 18 | 4-NHAlloc | HFIP | 0.5 h | 98% |

| | | | | |
|---|---|---|---|---|
| a Yield after chromatography | | | | |

### Product data:

Compound 9: mp 71-72°C; ¹H NMR (CDCl₃), 7.04 - 7.16 (m, 2H), 6.54 - 6.67 (m, 2H), 3.65 (broad s., 2H); ¹³C NMR (CDCl₃) 144.92, 129.10, 123.14, 116.21; MS ESI m/z (%) 128 (M+H+,100%); HRMS ESI m/z (M+H+) 128.02576. Calc 128.02615.
Compound 10: mp 63-64°C; ¹H NMR (CDCl₃), 7.33 (d, J=8.67 Hz, 1H), 6.79 (d, J=2.64 Hz, 1H), 6.45 (dd, J=8.67, 2.64 Hz, 1H), 3.74 (broad s, 2H); ¹³C NMR (CDCl₃) 146.64, 134.62, 133.88, 116.38, 114.92, 109.75; MS ESI m/z (%) 205 (M+H+,53%); HRMS ESI m/z (M+H+) 205.93645. Calc 205.93667.
Compound 11: mp 147-148°C; ¹H NMR (CDCl₃), 8.08 (d, J=9.04 Hz, 2H), 6.63 (d, J=9.04 Hz, 2H), 4.41 (broad s, 2H); ¹³C NMR (CDCl₃) 153.41, 138.37, 126.34, 113.16; MS EI m/z (%) 138 (M+,47%); Anal. calc. for C₆H₆N₂O₂: C, 52.17; H, 4.38; N, 20.28. Found: C, 52.50; H, 4.40; N, 19.99.
Compound 12: mp 59-60°C; ¹H NMR (CDCl₃), 6.72 - 6.79 (m, 2H), 6.63 - 6.70 (m, 2H), 3.76 (s, 3H), 3.43 (broad s, 2H); ¹³C NMR (CDCl₃) 152.78, 139.90, 116.40, 114.78, 55.72; MS ESI m/z (%) 124 (M+H+,100%); HRMS ESI m/z (M+H+) 124.07530. Calc 124.07569.
Compound 13: Oil; ¹H NMR (CDCl₃), 7.08 (t, J=8.10 Hz, 1H), 6.21 - 6.39 (m, 3H), 3.78 (s, 3H), 3.68 (broad s, 2H); ¹³C NMR (CDCl₃) 160.72, 147.78, 130.10, 107.90, 103.92, 101.04, 55.07; MS ESI m/z (%) 124 (M+H+,100%); HRMS ESI m/z (M+H+) 124.07526. Calc 124.07569.
Compound 14: mp 56-57°C; ¹H NMR (CDCl₃), 7.63 (dd, J=7.91, 1.32 Hz, 1H), 7.04 - 7.19 (m, 1H), 6.75 (dd, J=8.10, 1.51 Hz, 1H), 6.38 - 6.54 (m, 1H), 4.07 (broad s, 2H); ¹³C NMR (CDCl₃) 146.71, 138.96, 129.31, 119.95, 114.70, 84.15; MS ESI m/z (%) 220 (M+H+,100%); HRMS ESI m/z (M+H+) 219.96147. Calc 219.96177.
Compound 15: mp 135-136°C; ¹H NMR (CDCl₃), 6.58 (s, 4H), 3.35 (broad s, 4H); ¹³C NMR (CDCl₃) 138.57, 116.70; MS ESI m/z (%) 109 (M+H+,100%); HRMS ESI m/z (M+H+) 109.07565. Calcd. 109.07602.
Compound 16: Oil; ¹H NMR (CDCl₃), 7.00 (t, J=8.01 Hz, 1H), 6.30 - 6.20 (m, 3H), 3.60 (broad s, 2H), 1.19 - 1.33 (m, 3H), 1.09 - 1.14 (d, J=6.78 Hz, 18H); ¹³C NMR (CDCl₃) 157.48, 148.03, 130.26, 110.76, 108.64, 107.40, 18.36, 13.48; MS ESI m/z (%) 266 (M+H+,100%); HRMS ESI m/z (M+H+) 266.19327. Calc 266.19347.
Compound 17: mp 86-87°C; ¹H NMR (CDCl₃), 7.30 - 7.46 (m, 5H), 7.16 (d, J=7.54 Hz, 2H), 6.61 - 6.69 (m, 2H), 6.51 (broad s, 1H), 5.19 (s, 2H), 3.57 (broad s, 2H); ¹³C NMR (DMSO-d6) 153.97, 144.70, 137.35, 128.76, 128.56, 128.32, 128.26, 120.59, 114.34, 65.66; MESI m/z (%) 243 (M+H+,100%); HRMS ESI m/z (M+H+) 243.11249. Calc 243.11280.
Compound 18: mp = 52-53°C; ¹H NMR (CDCl₃), 7.16 (d, J=7.91 Hz, 2H), 6.69 - 6.59 (m, 2H), 6.50 (broad s, 1 H), 6.08 - 5.87 (m, 1H), 5.41 - 5.30 (m, 1H), 5.29 - 5.20 (m, 1H), 4.57 - 4.73 (m, 2H), 3.58 (broad s, 2H); ¹³C NMR (DMSO-d6) 153.82, 144.70, 134.00, 128.33, 120.66, 117.57, 114.32, 64.61; MS ESI m/z (%) 193 (M+H+,100%); HRMS ESI m/z (M+H+) 193.09683. Calc 193.09715.

### Example 4

To extend the synthetic potential of this deprotection method, this protocol was further expanded to a wide range of N-Boc amines in HFIP. In each case (except as otherwise noted), 1 mmol of protected compound was microwave heated at 150°C in HFIP (5 mL) for the time shown, and the deprotected compound recovered by chromatography. The results are shown in Table 4. In all cases, the deprotection product was obtained in good to excellent yields.

**Table 4: Deprotection of Amines in HFIP**

| **Protected Compound** | **Time** | **Product, No.** | **Yield^{a}** |
|---|---|---|---|
| 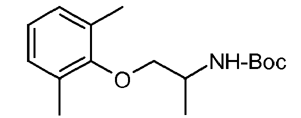 | 2h | 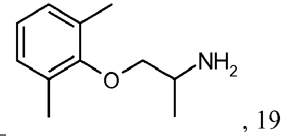 | 81% |
| 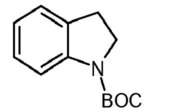 | 2h | 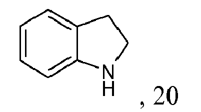 | 85% |
| 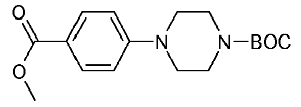 | 5 h | 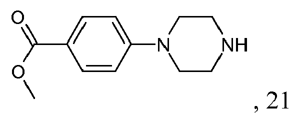 | 91% |
| 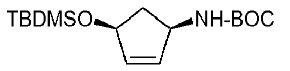 | 1 h | 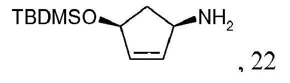 | 98% |
| 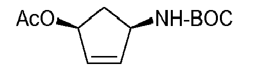 | 4 h^{b} | 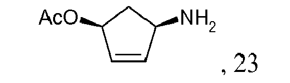 | 85% |
| 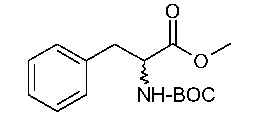 | 0.5 h | 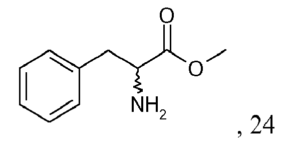 | 89% |
| 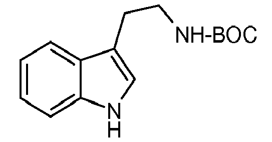 | 1 h | 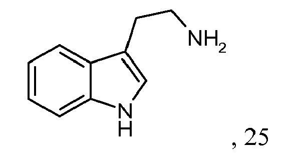 | 88% |
| 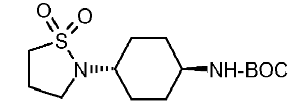 | 1 h | 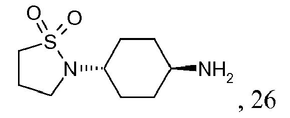 | >95%^{c} |

| | | | |
|---|---|---|---|
| a Yield after chromatography b Temperature = 100°C to avoid side products and low yield c Crude yield (pure by NMR) | | | |

### Product data:

Compound 19: Oil; ¹H NMR (CDCl₃), 7.06 - 6.86 (m, 3H); 3.59 - 3.51 (m, 1H), 3.70 - 3.62 (m, 1H), 3.46 - 3.29 (m, 1H), 2.30 (s, 6H), 1.72 (broad s, 2H), 1.18 (d, J=6.78 Hz, 3H); ¹³C NMR (CDCl₃) 155.49, 130.81, 128.88, 123.83, 78.23, 47.29, 19.78, 16.32; MS ESI m/z (%) 180 (M+H+,100%); HRMS ESI m/z (M+H+) 180.13782. Calc 180.13829.
Compound 20: Oil; ¹H NMR (CDCl₃), 7.14 (d, J=6.78 Hz, 1H), 7.04 (t, J=7.72 Hz, 1H), 6.77 - 6.70 (m, 1H), 6.67 (d, J=7.91 Hz, 1H), 3.57 (t, J=8.48 Hz, 2H), 3.05 (t, J=8.29 Hz, 2H); ¹³C NMR (CDCl₃) 151.57, 123.31, 127.19, 124.62, 118.64, 109.43, 47.32, 29.82; MS ESI m/z (%) 120 (M+H+,100%); HRMS ESI m/z (M+H+) 120.08030. Calc 120.08078.
Compound 21: mp = 104-105°C; ¹H NMR (CDCl₃), 7.92 (d, J=9.04 Hz, 2H), 6.87 (d, J=9.04 Hz, 2H), 3.87 (s, 3H), 3.34 - 3.23 (m, 4H), 3.08 - 2.94 (m, 4H), 1.74 (s, 1H); ¹³C NMR (CDCl₃) 167.15, 154.56, 131.18, 119.67, 113.60, 51.65, 54.16, 48.65, 47.50, 45.89; MS ESI m/z (%) 221 (M+H+,100%); HRMS ESI m/z (M+H+) 221.12805. Calc 221.12845.
Compound 22: Oil; ¹H NMR (CDCl₃), 5.86 - 5.81 (m, 1H), 5.80 - 5.74 (m, 1H), 4.75 - 4.66 (m, 1H), 3.78 - 3.69 (m, 1H), 2.72 - 2.60 (m, 1H), 1.96 (broad s, 2H), 1.38 - 1.22 (m, 1H), 0.90 (s, 9H), 0.09 (s, 6H); ¹³C NMR (CDCl₃) 137.92, 135.57, 76.37, 56.50, 45.88, 26.32, 18.19, -4.21; MS ESI m/z (%) 214 (M+H+,25%); HRMS ESI m/z (M+H+) 214.16183. Calc 214.16217.
Compound 23: Oil; ¹H NMR (CDCl₃), 6.04 - 5.97 (m, 1H), 5.88 - 5.79 (m, 1H), 5.58 - 5.47 (m, 1H), 3.93 - 3.78 (m, 1H), 2.86 - 2.69 (m, 1H), 2.05 (s, 3H), 1.62 (broad s, 2H), 1.48 - 1.32 (m, 1H); ¹³C NMR (CDCl₃) 170.79, 141.32, 130.67, 78.46, 56.49, 41.80, 21.65; MS ESI m/z (%) 142 (M+H+,100%); HRMS ESI m/z (M+H+) 142.08592. Calc 142.08626.
Compound 24: Oil; ¹H NMR (CDCl₃), 7.10 - 7.34 (m, 5H), 3.75-3.71(dd, J= ,1H), 3.71 (s, 3H), 3.08 (dd, J=13.38, 5.09 Hz, 1H), 2.84 (dd, J=13.56, 7.91 Hz, 1H), 1.52 (broad s, 2H); ¹³C NMR (CDCl₃), 175.34, 137.12, 129.16, 128.47, 126.73, 55.73, 51.87, 41.00; MS ESI m/z (%) 180 (M+H+,100%); HRMS ESI m/z (M+H+) 180.10156. Calc 180.10191.
Compound 25: mp = 111-112°C; ¹H NMR (CDCl₃), 8.35 (broad s, 1H), 7.64 (d, J=7.91 Hz, 1H), 7.44 - 7.32 (m, 1H), 7.26 - 7.18 (m, 1H), 7.18 - 7.09 (m, 1H), 7.04 (d, J=2.26 Hz, 1H), 1.36 (broad s, 2H), 3.11 - 3.01 (m,2H), 2.99 - 2.86 (m, 2H); ¹³C NMR (CDCl₃) 136.44, 127.49, 122.06, 121.96, 119.21, 118.87, 113.69, 111.16, 42.34, 29.49; MS ESI m/z (%) 161 (M+H+,100%); HRMS ESI m/z (M+H+) 161.10693. Calc 161.10732.
Compound 26: Oil; ¹H NMR (CDCl₃), 3.34 - 3.48 (m, 1H), 3.27 (t, J=6.78 Hz, 2H), 3.12 (t, 2H), 2.55 - 2.69 (m, 1H), 2.25 - 2.40 (m, 2H), 1.85 - 2.02 (m, 4H), 1.45 - 1.62 (m, 2H), 1.36 (broad s, 2H), 1.14 - 1.30 (m, 2H); ¹³C NMR (CDCl₃) 52.56, 49.75, 47.07, 41.54, 35.50, 29.41, 18.70; MS ESI m/z (%) 219 (M+H+,100%); HRMS ESI m/z (M+H+) 219.11609. Calc 219.11618.

### Example 5

In general, it was found that HFIP is a more reactive solvent than TFE for N-BOC deprotection reactions. Thus, the use of HFIP over TFE on the same substrate under similar conditions consistently reduced reaction times (see Examples 2 and 3 above). On the basis of the reactivity differences between TFE and HFIP, 4-N-BOC-4-(4-BOC-piperazin-1-yl)-indole was sequentially deprotected. As set forth in the Scheme below, TFE (microwave heating for 2 h at 150°C) was used to selectively remove the indole BOC moiety in good yields (80%). Further treatment of the partially-deprotected compound with HFIP (microwave heating for 2 h at 150°C) efficiently completed the cleavage of the remaining N-BOC group on the piperazine ring (yield = 81 %). On the other hand, if selectivity is not required both BOC groups can be removed simultaneously using HFIP as a solvent.

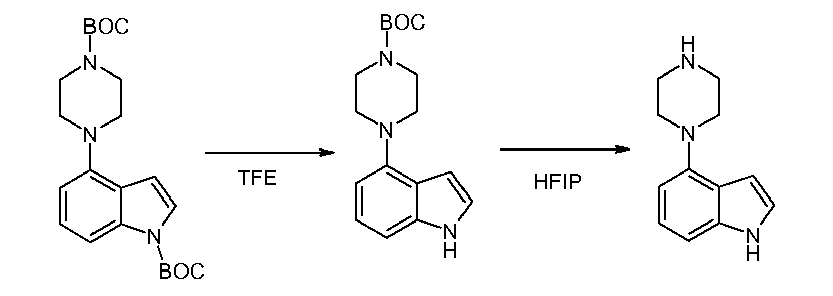

4-(1H-Indol-4-yl)-piperazine-1-carboxylic acid t-butyl ester: mp = 139 - 140°C; ¹H NMR (CDCl₃), 8.27 (broad s, 1H), 7.25-7.07 (m, 3H), 6.60-6.54 (m, 2H), 3.67 (t, 4H), 3.19 (t, 4H), 1.50 (s, 9H); ¹³C NMR (CDCl₃), 154.94, 145.53, 136.97, 122.87, 122.66, 121.36, 106.87, 106.18, 100.94, 76.60, 51.31, 43.69, 28.47; MS ESI m/z (%) 302 (M+H+,100%); HRMS ESI m/z (M+H+) 302.18616. Calcd. 302.18630. 4-Piperazin-1-yl-1H-indole: mp = 198 - 199°C (dec.); ¹H NMR (CDCl₃), 8.37 (broad s, 1H), 7.19-7.03 (m, 3H), 6.67 - 6.51 (m, 2H), 3.24-3.18 (m, 4H), 3.19-3.05 (m, 4H), 2.01 (broad s, 1H); ¹³C NMR (DMSO-d6), 145.99, 136.96, 123.25, 121.57, 120.78, 105.75, 105.30, 99.93, 52.28, 45.98; MS ESI m/z (%) 202 (M+H+,100%); HRMS ESI m/z (M+H+) 202.13351. Calc 202.13387.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective spirit and scope of the present invention. All such modifications are intended to be within the scope of the claims appended hereto.

## Claims

1. A method for deprotecting a compound having BOC-protected nitrogen atoms, said method comprising:
a) dissolving a compound having a BOC-protected nitrogen atom in a fluorinated alcohol to form a solution;
b) heating said solution for a period of time sufficient to remove BOC from said BOC-protected nitrogen, thereby providing a deprotected compound.

2. The method of claim 1, wherein said heating comprises heating by microwave radiation.

3. The method of claim 1 or 2, further comprising:
c) recovering said deprotected compound from said solution.

4. The method of claims 1 to 3, wherein said fluorinated alcohol is selected from the group consisting of 2,2,2-trifluoroethanol and 1,1,1,3,3,3-hexafluoroisopropanol.

5. A method for sequentially removing BOC protecting groups from nitrogen atoms having different lability in the same compound using first and second fluorinated alcohol solvents having different reactivities, comprising:
a) dissolving a compound comprising first and second BOC-protected nitrogen atoms having different labilities in a first fluorinated alcohol to form a first solution;
b) heating the first solution to remove BOC from said first BOC-protected nitrogen atom, providing a partially-deprotected compound;
c) dissolving said partially-deprotected compound in a second fluorinated alcohol solvent having higher reactivity than said first fluorinated alcohol solvent, to form a second solution; and
d) heating said second solution to remove BOC from said second BOC-protected nitrogen atom.

6. The method of claim 5, wherein said first fluorinated alcohol comprises 2,2,2-trifluoroethanol.

7. The method of claim 6, wherein said second fluorinated alcohol comprises 1,1,1,3,3,3-hexafluoropropan-2-ol.
